# EUROPÄISCHE PATENTSCHRIFT

(11) **EP 1 880 764 B1**
(45) Veröffentlichungstag und Bekanntmachung des Hinweises auf die Patenterteilung: **05.09.2012**
(21) Anmeldenummer: 06015167.7
(22) Anmeldetag: 20.07.2006
(51) Int. Cl.: C12M 3/00, G02B 21/34, B01L 3/00, C12M 1/18

(54) **Probenträger zur Untersuchung von Zellwachstum**
Sample carrier for examining cell growth
Porte échantillons destiné à l'analyse de la croissance cellulaire

(43) Veröffentlichungstag der Anmeldung: 23.01.2008
(73) Patentinhaber: ibidi GmbH, 82152 Martinsried (DE)
(72) Erfinder: Zantl, Roman, 85598 Baldham (DE); Kahl, Johan-Valentin, 80797 München (DE)
(74) Vertreter: Grünecker, Kinkeldey, Stockmair & Schwanhäusser

(56) Entgegenhaltungen:
- EP-A- 1 609 851
- EP-A2- 1 079 224
- WO-A-2004/071661
- WO-A2-03/036265
- US-A- 5 229 163
- US-A1- 2005 101 010
- US-B1- 6 312 952

## Beschreibung

Die Erfindung betrifft einen Probenträger zur Untersuchung von Zellwachstum, insbesondere für Angiogeneseuntersuchungen.

In der Zellbiologie werden Zellwachstumsuntersuchungen häufig durchgeführt, indem ein Trägermaterial, wie beispielsweise ein Kollagengel wie das BD Matrigel der Firma Becton, Dickinson and Company, in Vertiefungen von 96-Well-, 48-Well- oder 24-Well-Platten gefüllt wird. Ein derartiges Trägermaterial wird im Allgemeinen auch als Matrix bezeichnet.

Auf einer solchen Matrix werden dann beispielsweise gefäßbildende Zellen, z.B. Human Umbilical Vein Endothelial Cells (HUVEC), ausgesät. Nach einer gewissen Zeit (bspw. Stunden oder Tage) zeigen die Zellen dann eine charakteristische, netzartig erscheinende Struktur, die nachweist, dass die Zellen in der Lage sind, beispielsweise Blutgefäße zu bilden. Die Zellstrukturen werden dann beispielsweise mit Mikroskopen, vorzugsweise mit Phasenkontrast, untersucht.

Derartige Untersuchungen werden insbesondere in der Tumorforschung eingesetzt, wobei es darum geht, die Gefäßneubildung durch pharmakologische und biotechnologische Methoden und Substanzen zu hemmen und damit das Tumorwachstum zu behindern. Da Tumore aufgrund ihres starken Wachstums einen sehr hohen Bedarf an Blutgasen und Nährstoffen haben, ist ihr Wachstum in besonderer Weise abhängig von einer guten Versorgung mit diesen und kann deswegen durch eine Behinderung der Gefäßbildung eingeschränkt werden.

Werden bei Zellwachstumsuntersuchungen die Vertiefungen herkömmliche Weil-Platten mit entsprechenden Trägermaterialien teilweise befüllt, erhält man auf einer entsprechenden Höhe der Vertiefungen jeweils eine Oberfläche des Trägermaterials. Aufgrund von Oberflächenspannungseffekten kann die Form der Oberfläche nicht im Voraus bestimmt werden. Da das Wachstum von danach in einem Nährmedium zugegebenen Zellen durch die Oberflächengeometrie des Trägermaterials beeinflusst wird, erfolgt dieses unkontrollierbar. Durch die unbekannte Oberflächengeometrie und das unkontrollierte Zellwachstum werden auch nachfolgende mikroskopische Untersuchungen erschwert.

Aus der WO 03/036265 ist ein Probenbehälter mit Teilbehälten bekannt, die in fluidische Verbindung gebracht werden können.

Angesichts des Nachteils des Standes der Technik ist es daher die Aufgabe der vorliegenden Erfindung, einen Probenträger zur Untersuchung von Zellwachstum bereitzustellen, durch den ein besser kontrolliertes Zellwachstum ermöglicht wird und anschließende mikroskopischen Untersuchungen mit hoher Genauigkeit und Flexibilität durchgeführt werden können. Diese Aufgabe wird gelöst durch den Gegenstand von Anspruch 1.

Erfindungsgemäß wird ein Probenträger zur Untersuchung von Zellwachstum bereitgestellt, umfassend
ein Substrat mit einem Reservoir mit einem Boden,
wobei das Reservoir bis zu einer vorherbestimmten Höhe der Seitenwand, die kleiner als die Gesamthöhe des Reservoirs ist, mit einem Trägermaterial für Zellwachstum befüllt ist, und
die Seitenwand des Reservoirs derart ausgebildet ist, dass ein vorherbestimmter Kontaktwinkel des Trägermaterials bezüglich der Seitenwand einstellbar ist.

Durch die Einstellbarkeit des Kontaktwinkels, d.h. des Winkels zwischen der Seitenwand und der Oberfläche des Trägermaterials, lässt sich wählen, ob und in welcher Stärke das Trägermaterial einen Meniskus ausbildet. Somit kann die Oberflächengeometrie genau eingestellt werden. Dies erlaubt insbesondere auch die Einstellung eines Kontaktwinkels von 90°, so dass das Trägermaterial eine plane Oberfläche aufweist. Auch Kontaktwinkel von größer 90° sind möglich, um eine nach oben gewölbte Oberfläche des Trägermaterials zu erhalten. Für manche Untersuchungen mag es von Vorteil sein, den Kontaktwinkel um die 90° einzustellen, da in diesem Fall zu untersuchende Zellen in einer Ebene wachsen würden, was starke Mikroskopvergrößerungen bei kleiner Tiefenschärfe erlaubt. Andererseits kann für manche Untersuchungen ein Meniskus mit einem Kontaktwinkel kleiner 90° erwünscht sein, da sich dann in Nährmedium hinzugegebene Zellen am tiefsten Punkt des Trägermaterials sammeln. Eine sehr flexible und genaue Anpassung der Oberflächengeometrie an anschließend durchzuführende Untersuchungen ist somit möglich.

Die Einstellbarkeit des Kontaktwinkels hat weiterhin den Vorteil, dass auch die Menge des verwendeten Trägermaterials genau dosiert werden kann. Falls insbesondere das Trägermaterial für bestimmte Untersuchungen eine Mindestdicke aufweisen muss, wird im Falle einer Meniskusbildung unnötiges Trägermaterial an den Seitenwänden bereitgestellt.

Bei einer derartigen Ausbildung der Seitenwand kann damit auch der Kontaktwinkel eines hinzugefügten Nährmediums, insbesondere auf Wasserbasis, eingestellt werden. So kann beispielsweise erreicht werden, dass zwar das Trägermaterial einen gewünschten Meniskus aufweist, das Nährmedium jedoch einen Kontaktwinkel von 90° und damit eine plane Oberfläche hat. Besonders bei geringen Brechungsindexunterschieden von Trägermaterial und Nährmedium kann auf diese Weise eine einfache Mikroskopierbarkeit über den gesamten mit Zellen besiedelten Bereich gewährleistet werden. Insbesondere ist es von Vorteil, wenn Grenzflächen mit relevanten Brechungsindexunterschieden (beispielsweise mit einem Unterschied von wenigstens 0,2) im Strahlengang eines Mikroskops senkrecht zur optischen Achse stehen. Wenn sich das Trägermaterial in seinem Brechungsindex von dem darüber angeordneten Nährmedium entsprechend unterscheidet, wird ebenfalls vorteilhafterweise für das Trägermaterial ein Kontaktwinkel von um die 90° gewählt.

Die Seitenwand des Reservoirs kann auf der vorherbestimmten Höhe eine Kante, insbesondere eine umlaufende Kante, derart aufweisen, dass der vorherbestimmte Kontaktwinkel des Trägermaterials bezüglich der Seitenwand einstellbar ist. Die Kante kann insbesondere durch einen Vorsprung gebildet sein. Bei einer Kante auf der vorherbestimmten Höhe ist Kontaktwinkel bezüglich der Seitenwand unterhalb der Kante, d.h. zwischen Boden und Kante, zu bestimmen.

Aufgrund der Kante kann auf der vorherbestimmten Höhe der Kontaktwinkel eingestellt werden. Bildet sich insbesondere beim Einfüllen des Trägermaterials ein Meniskus aus, so bleibt dieser Meniskus vorhanden, solange das Trägermaterial an den Seiten noch nicht die Kante erreicht hat. Sobald die Kante erreicht ist und weiter Trägermaterial hinzugegeben wird, steigt das Trägermaterial an den Seitenwänden aufgrund der Oberflächenspannung an der Kante nicht weiter an; statt dessen vergrößert sich der Kontaktwinkel bzw. verringert sich der Meniskus bei weiterem Hinzugeben von Trägermaterial kann der Kontaktwinkel bis auf über 90° erhöht werden, solange die Oberflächenspannung des Trägermaterials verhindert, dass das Trägermaterial an den Seitenwänden über die Kante und die vorherbestimmte Höhe ansteigt.

Die Seitenwand des Reservoirs kann auf der vorherbestimmten Höhe einen Absatz, insbesondere einen umlaufenden Absatz, aufweisen, wobei das Reservoir parallel zum Boden unmittelbar unterhalb der vorherbestimmten Höhe eine erste Querschnittsfläche und unmittelbar oberhalb der vorherbestimmten Höhe eine zweite Querschnittsfläche aufweisen, wobei die zweite Querschnittsfläche größer als die erste Querschnittsfläche ist.

Durch einen Absatz kann beispielsweise eine rechtwinklige Kante gebildet werden. Der Absatz erlaubt es, den Kontaktwinkel des Trägermaterials einzustellen, da das Trägermaterial, aufgrund der Kante beim Absatz, bei Erreichen des Absatzes an der Seitenwand wegen der Oberflächenspannung zunächst nicht weiter steigt, sondern der Meniskus verringert wird.

Grundsätzlich können die Abschnitte des Reservoirs unterhalb und oberhalb der vorherbestimmten Höhe beliebige und verschiedene Formen annehmen. Sie können beispielsweise konisch ausgebildet sein.

Insbesondere kann das Reservoir parallel zum Boden vom Boden bis zu der vorherbestimmten Höhe eine erste Querschnittsfläche und von der vorherbestimmten Höhe bis zu einer weiteren vorherbestimmten Höhe eine zweite Querschnittsfläche aufweisen, wobei die zweite Querschnittsfläche größer als die erste Querschnittsfläche ist.

Damit sind beide Abschnitte des Reservoirs (zwischen Boden und (erster) vorherbestimmter Höhe und zwischen (erster) vorherbestimmter Höhe und weiterer vorherbestimmter Höhe) zylindrisch ausgebildet. Der somit gebildete Absatz weist eine rechtwinklige Kante auf. Die Querschnittsfläche kann dabei beispielsweise kreisförmig oder rechteckig ausgebildet sein. Bei diesen Ausführungen muss die weitere vorherbestimmte Höhe jedoch nicht der Gesamthöhe des Reservoirs entsprechen.

Bei den zuvor beschriebenen Probenträgern kann das Größenverhältnis von zweiter zu erster Querschnittsfläche wenigstens 1,2, vorzugsweise wenigstens 1,8, weiter bevorzugt 2 - 4, betragen. Mit diesen Größenverhältnissen wird in besonders vorteilhafter Weise eine Kante ausgebildet, die ein Einstellen eines vorherbestimmten Kontaktwinkels bei einem Befüllen des Reservoirs bis zu der (ersten) vorherbestimmten Höhe erlaubt.

Insbesondere kann die erste Querschnittsfläche eine Fläche von 0,8 mm² - 175 mm², vorzugsweise 0,8 - 65 mm², aufweisen. Die erste und/oder zweite Querschnittsfläche kann elliptisch oder kreisförmig ausgebildet sein. Durch das Fehlen von auf den Boden zulaufende Kanten wird das Totvolumen verringert und werden unerwünschte Oberflächenspannungseffekte vermieden.

Bei den zuvor beschriebenen Probenträgern kann die vorherbestimmte Höhe 50 µm - 1 mm, vorzugsweise 50 - 250 µm, betragen. Ein Trägermaterial in einem solchen Dickenbereich erlaubt zum einen in vorteilhafter Weise ein Wachstum von ausgesäten Zellen und zum Anderen eine gute Beobachtung mittels Mikroskopieverfahren.

Der Boden der zuvor beschriebenen Probenträger kann eine Dicke von 1 µm - 2 mm, vorzugsweise 10 µm - 250 µm, aufweisen. Damit sind insbesondere Untersuchungen mittels inverser Mikroskopie besonders gut möglich.

Der Boden der zuvor beschriebenen Probenträger kann ein optisch transparentes Material, insbesondere Glas oder einen Kunststoff, wie COC (Cyclo-Olefin-Copolymer), COP (Cyclo-Olefin-Polymer), PMMA (Polymethylmethacrylat), PC (Polycarbonat), PE (Polyethylen) oder PS (Polystyrol) umfassen.

Mit diesen Materialien kann der Probenträger in einfacher Weise hergestellt und dann für die inverse Mikroskopie verwendet werden. Bei den zuvor beschriebenen Probenträgern kann der Boden für sichtbares Licht im Wesentlichen keine Eigenfluoreszenz und/oder einen Brechungsindex größer als 1,2 und/oder kleiner als 1,7 aufweisen.

Im Wesentlichen keine Eigenfluoreszenz bedeutet, dass die Eigenfluoreszenz kleiner oder gleich der Eigenfluoreszenz eines herkömmlichen Deckglases (beispielsweise reinweißes Glas der hydrolytischen Klasse 1, wie Menzel-Deckglas, insbesondere mit der Stärke Nr. 1,5) ist. Derartige optische Eigenschaften sind insbesondere bei der inversen Mikroskopie von großem Vorteil.

Bei den oben beschriebenen Probenträgern kann das Trägermaterial ein Gel, insbesondere ein vernetztes oder polymerisiertes Gel, Silikon oder ein Polymer, insbesondere ein vernetztes Polymer, umfassen. Diese Materialien sind in besonders vorteilhafter Weise für das Wachsen von Zellen auf ihrer Oberfläche geeignet.

Bei den zuvor beschriebenen Probenträgern kann der vorherbestimmte Kontaktwinkel derart eingestellt sein, dass die Differenz zwischen dem Kontaktwinkel und dem Neigungswinkel der Seitenwand bezüglich des Bodens -45° bis +45°, vorzugsweise -20° bis +20°, weiter bevorzugt -10° bis +10°, insbesondere 0°, beträgt.

Bei einer senkrecht zum Boden stehenden Seitenwand zwischen Boden und vorherbestimmter Höhe bedeutet eine Differenz von 0° zwischen Kontaktwinkel und Neigungswinkel, dass die Oberfläche des Trägermaterials plan ist und parallel zum Boden verläuft. Ist die Differenz negativ, ist ein Meniskus ausgebildet, während bei einer positiven Differenz das Trägermaterial nach oben gewölbt ist.

Je nach Art der durchzuführenden Untersuchung kann eine andere Wahl des Kontaktwinkels von Vorteil sein. Bei einer planen Oberfläche des Trägermaterials parallel zum Boden werden ausgesäte Zellen im Wesentlichen in einer Ebene wachsen, so dass hier Mikroskopie und Untersuchungen mit starken Vergrößerungen und geringer Tiefenschärfe ermöglicht werden. Die Ausbildung eines Meniskus kann erwünscht sein, wenn sich die Zellen insbesondere beim Aussäen in einem kleinen Bereich, um den tiefsten Punkt der Oberfläche des Trägermaterials herum, sammeln sollen.

Bei der Einstellung des Kontaktwinkels ist weiterhin zu berücksichtigen, dass Grenzflächen mit signifikanten Brechungsindexunterschieden vorteilhafterweise senkrecht zum optischen Strahlengang eines Mikroskops oder parallel zum Boden des Reservoirs angeordnet sein können. Ein Brechungsindexunterschied ist insbesondere signifikant, wenn er größer als 0,2, insbesondere größer als 0,3, ist. Somit ist eine Differenz von 0° zwischen Kontaktwinkel und Neigungswinkel insbesondere beim Einsatz von Trägermaterialien, die zum Nährmedium einen signifikanten Brechungsindexunterschied aufweisen, von Vorteil, da dann die Grenzfläche zwischen Trägermaterial und Nährmedium parallel zum Boden und senkrecht zur optischen Achse ausgebildet ist.

Bei den zuvor beschriebenen Probenträgern kann die Seitenwand an vorherbestimmten Bereiche hydrophil und/oder hydrophob ausgebildet sein. Auf diese Weise wird ebenfalls ermöglicht, den Kontaktwinkel einzustellen bzw. zu beeinflussen. Hydrophile oder hydrophobe Bereiche können beispielsweise durch geeignete Beschichtungen oder Oberflächenbehandlungen erzeugt werden.

Das Substrat der zuvor beschriebenen Probenträger kann einen Kunststoff umfassen. Insbesondere kann bei den zuvor beschriebenen Probenträgern das Substrat eine Kunststofffolie umfassen, durch die der Boden des Reservoirs gebildet wird. Das Substrat kann insbesondere genau einen Kunststoff umfassen.

Alternativ kann das Substrat der zuvor beschriebenen Probenträger einstückig, insbesondere als Spritzgussteil, ausgebildet sein. Damit lässt sich der Probenträger in besonders einfacher Weise herstellen.

Die zuvor beschriebenen Probenträger können weiterhin einen Deckel zum Verschließen des Reservoirs umfassen. Die dem Reservoir zugewandte Oberfläche und/oder die dem Reservoir abgewandte Oberfläche des Deckels können dabei insbesondere plan und parallel zum Boden des Reservoirs ausgebildet sein. Dadurch wird ermöglicht, dass die Grenzfläche des Deckels zu Luft bzw. zu einem in das Reservoir eingebrachten Nährmedium senkrecht zu einer optischen Achse ausgerichtet ist.

Weitere Vorteile und Merkmale der Erfindung werden nachfolgend anhand der Figuren erläutert. Dabei zeigt
- Fig. 1: einen zweistückig ausgebildeten Probenträger, bei dem Trägermaterial und Nährmedium bis zu der vorherbestimmten Höhe aufgefüllt sind;
- Fig. 2: einen zweistückig ausgebildeten Probenträger, bei dem ein Nährmedium bis zur Gesamthöhe des Reservoirs aufgefüllt ist;
- Fig. 3: einen einstückigen Probenträger, bei dem das Trägermaterial eine Oberfläche parallel zum Reservoirboden aufweist;
- Fig. 4: einen einstückigen Probenträger, bei dem das Nährmedium bis oberhalb der vorherbestimmten Höhe, aber nicht bis zur Gesamthöhe des Reservoirs aufgefüllt ist;
- Fig. 5: einen zweistückig ausgebildeten Probenträger mit einer teilweise hydrophilisierten Oberfläche.

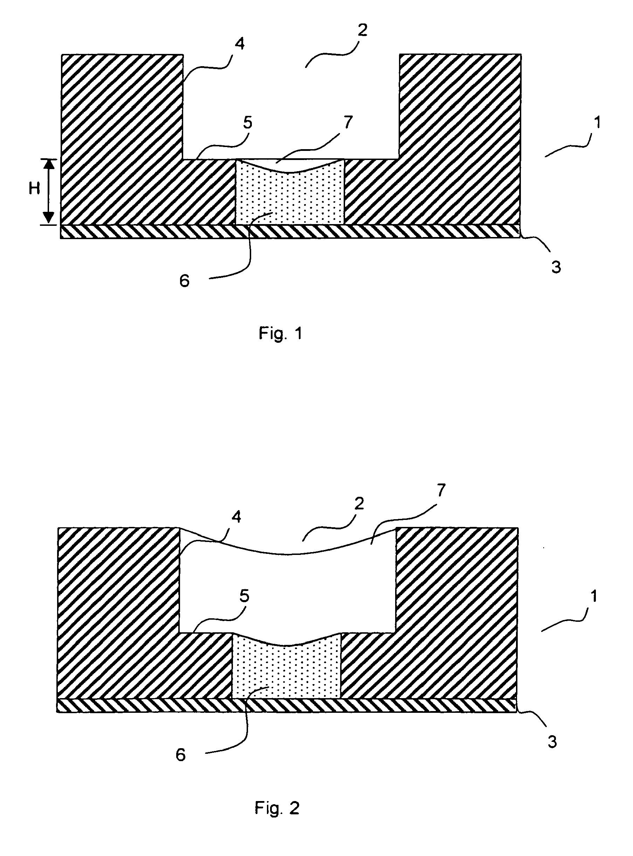

In Fig. 1 ist ein Probenträger mit einem Substrat 1 in Querschnittsansicht schematisch dargestellt, wobei in dem Substrat 1 ein Reservoir 2 ausgebildet ist. Das Substrat 1 umfasst eine Kunststofffolie 3, durch die der Boden des Reservoirs 2 gebildet ist. Das Reservoir weist zwei zylindrische Abschnitte auf. Der erste Abschnitt reicht vom Boden bis zu einer vorherbestimmten Höhe H. Auf dieser Höhe weist die Seitenwand 4 des Reservoirs einen Absatz 5 auf, durch den eine umlaufende rechtwinklige Kante gebildet wird. Der zweite Abschnitt des Reservoirs reicht von der vorherbestimmten Höhe H bis zur Gesamthöhe des Reservoirs.

In dem gezeigten Ausführungsbeispiel ist die Querschnittsfläche des ersten Abschnitts bis zur Höhe H kleiner als die Querschnittsfläche des zweiten Abschnitts unmittelbar oberhalb der vorherbestimmten Höhe H.

Der erste Abschnitt des Reservoirs ist bis zu der vorherbestimmten Höhe H mit einem Trägermaterial 6 befüllt; d.h., das Trägermaterial reicht an seinem höchsten Punkt (an der Seitenwand) bis zur Höhe H. Bei dem Trägermaterial kann es sich beispielsweise um ein Kollagengel wie das Produkt BD Matrigel der Firma Becton, Dickinson and Company handeln.

Aufgrund der durch den Absatz auf Höhe H gebildete Kante wird ermöglicht, dass der Kontaktwinkel des Trägermaterials 6 bezüglich der Seitenwand 4 einstellbar ist. Bei einem Befüllen des ersten Abschnitts mit dem Trägermaterial bildet sich zunächst ein Meniskus aus. Dieser bleibt bestehen, bis das Trägermaterial an der Seitenwand die Höhe H erreicht hat. An der Kante des Vorsprungs steigt das Trägermaterial bei weiterem Befüllen aufgrund der Oberflächenspannung nicht weiter an; statt dessen verringert sich der Kontaktwinkel und damit der Meniskus. In dem gezeigten Beispiel ist der Kontaktwinkel bezüglich der Seitenwand kleiner als 90°; dies bedeutet, dass die Differenz zwischen dem Kontaktwinkel und dem Neigungswinkel der Seitenwand bezüglich des Bodens, wobei der Neigungswinkel in dem gezeigten Beispiel 90° beträgt, negativ ist.

Das Trägermaterial wird üblicherweise in flüssiger Form eingefüllt und härtet dann aus. Eine Beschleunigung des Aushärtens kann durch beispielsweise durch Erwärmen des Trägermaterials erreicht werden. Damit wird gegebenenfalls verhindert, dass das Trägermaterial nach dem Einfüllen während des Aushärtens über die Kante des Absatzes kriecht.

In die durch den Meniskus des Trägermaterials 6 gebildete Vertiefung ist ein Nährmedium 7, das Zellen enthält, gefüllt worden. Auch im Falle des Befüllens mit Nährmedium bildet dieses zunächst einen Meniskus aus, der sich bei weiterem Befüllen aufgrund der Kante des Absatzes jedoch verringert, so dass, wie in dem gezeigten Beispiel, ein Kontaktwinkel des Nährmediums bezüglich der Seitenwand von 90° eingestellt werden kann. Damit ist die Grenzfläche zwischen Nährmedium 7 und Luft plan und parallel zum Boden. Sie ist insbesondere auch senkrecht zu der optischen Achse bei Durchführung einer Untersuchung mittels inverser Mikroskopie.

Die dargestellte Konfiguration ist besonders von Vorteil, wenn die Brechungsindexdifferenz zwischen Trägermaterial 6 und Nährmedium 7 klein ist, beispielsweise kleiner als 0,2, so dass in erster Linie die Grenzflächen zwischen Nährmedium und Luft bzw. zwischen Trägermaterial und Reservoirboden für die Mikroskopieruntersuchung von Bedeutung sind. Allerdings ist zu berücksichtigen, dass bei Ausbildung eines Meniskus durch das Trägermaterial die Zellen nicht in einer Ebene wachsen, so dass bei einer mikroskopischen Untersuchung eine hinreichende Tiefenschärfe bereitgestellt werden muss.

Insbesondere bei Untersuchungen mittels inverser Mikroskopie ist es erforderlich, dass der Boden 3 optisch transparent ist und gegebenenfalls weitere vorherbestimmte optische Eigenschaften, wie beispielsweise eine geringe Eigenfluoreszenz, aufweist. Weiterhin ist auch eine geringe Bodendicke für mikroskopische Untersuchungen von Vorteil. Dies lässt sich insbesondere erreichen, indem der Boden durch eine Kunststofffolie, beispielsweise mit einer Dicke von zwischen 10 und 250 µm, gebildet wird, die mit dem anderen Teil des Substrats beispielsweise durch Verkleben oder Ultraschallbonden verbunden wird.

Die in Fig. 1 gezeigte Geometrie des Reservoirs mit zwei Abschnitten unterschiedlicher Querschnittsflächen hat den weiteren Vorteil, dass auch größere Mengen an Nährmedium hinzugegeben werden können, ohne dass dieses unkontrolliert überläuft und den Probenträger verunreinigt. Ein Fall mit einer größeren Menge an Nährmedium ist schematisch in der Querschnittsansicht von Fig. 2 illustriert. Hier ist das Nährmedium 7 bis zur Gesamthöhe des Reservoirs aufgefüllt worden, wobei in dem gezeigten Beispiel noch ein Meniskus in dem Nährmedium vorhanden ist.

Der Probenträger kann weiterhin einen Deckel umfassen, der auf das Substrat aufgebracht wird und das Reservoir verschließt.

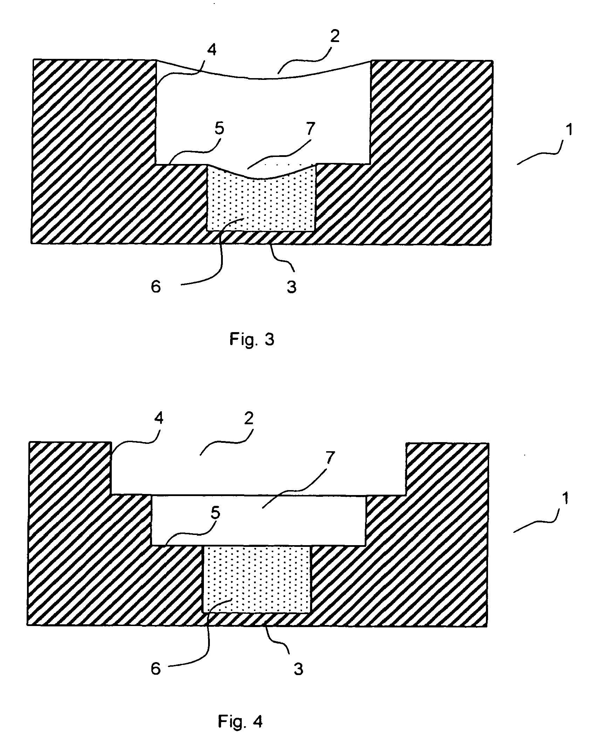

In Fig. 3 ist in schematischer Querschnittsansicht ein einstückig ausgebildetes Substrat 1 eines Probenträgers gezeigt. Die Geometrie des Reservoirs entspricht der in den Figuren 1 und 2 gezeigten Form. Bei Fig. 3 ist allerdings das Trägermaterial 6 bis zum Absatz derart aufgefüllt worden, dass der Kontaktwinkel bezüglich der Seitenwand 90° bzw. die Differenz zwischen Kontaktwinkel und Neigungswinkel der Seitenwand 0° beträgt. Damit wurde die Oberfläche des Trägermaterials plan eingestellt. Dies hat insbesondere den Vorteil, dass ausgesäte Zellen auf dem Trägermaterial in einer Ebene wachsen, so dass keine besonders große Tiefenschärfe bei Mikroskopieuntersuchungen erforderlich ist. Durch den zweiten Abschnitt des Reservoirs oberhalb der vorherbestimmten Höhe kann trotz der planen Trägermaterialoberfläche eine größere Menge an Nährmedium mit zu untersuchenden Zellen hinzugegeben werden.

Das einstückige Substrat kann insbesondere ein Spritzgussteil sein, was eine einfache Herstellung ermöglicht.

In Fig. 4 ist ein weiteres Ausführungsbeispiel eines einstückigen Substrats 1 eines Probenträgers gezeigt. In diesem Beispiel weist das Reservoir drei Abschnitte auf; einen ersten, der bis zu der vorherbestimmten Höhe, zu der das Trägermaterial eingefüllt wird, reicht, einen zweiten Abschnitt bis zu einer weiteren vorherbestimmten Höhe, sowie einen dritten Abschnitt bis zur Gesamthöhe des Reservoirs.

Die Querschnittsflächen vergrößern sich von Abschnitt zu Abschnitt, wobei jeder Abschnitt zylindrisch ausgebildet ist, so dass auf der (ersten) vorherbestimmten Höhe und auf der weiteren vorherbestimmten Höhe ein Absatz gebildet wird, der das Einstellen eines vorherbestimmten Kontaktwinkels an der jeweiligen Kante ermöglicht. Dabei wird auf der ersten vorherbestimmten Höhe der Kontaktwinkel des Trägermaterials und auf der weiteren vorherbestimmten Höhe der Kontaktwinkel des Nährmediums jeweils bezüglich der Seitenwand eingestellt.

Durch den dritten Abschnitt zwischen der weiteren vorherbestimmten Höhe und der Gesamthöhe des Reservoirs wird erreicht, dass bei einem Überfließen des Nährmediums nicht der gesamte Probenträger verunreinigt wird. In dem in Fig. 4 gezeigten Beispiel wurden die Kontaktwinkel des Trägermaterials und des Nährmediums derart eingestellt, dass die Grenzflächen zwischen dem Trägermaterial und Nährmedium bzw. zwischen Nährmedium und Luft plan und parallel zum Boden ausgebildet sind.

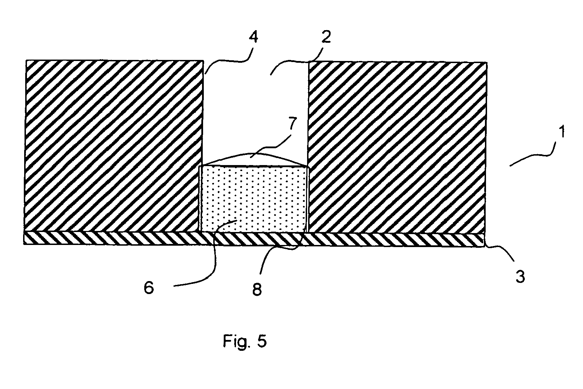

Fig. 5 zeigt schematisch ein weiteres Ausführungsbeispiel, bei dem die Seitenwand 4 bis zu der vorherbestimmten Höhe H einen hydrophilen Bereich 8 aufweist. Dieser Bereich kann beispielsweise durch eine Plasmabehandlung erhalten werden, bei der der obere, nicht zu behandelnde Abschnitt der Seitenwand 4, abgedeckt wurde. Ein derartiger hydrophiler Abschnitt 8 ermöglicht es ebenfalls, dass das Reservoir 2 bis zu der vorherbestimmten Höhe H mit Trägermaterial derart aufgefüllt wird, das der Kontaktwinkel 90° beträgt. In dem gezeigten Beispiel beträgt der Kontaktwinkel zwischen Nährmedium 7 und Seitenwand 4 mehr als 90°.

Die in den zuvor erläuterten Beispielen genannten Merkmale können allerdings auch in anderer Weise miteinander kombiniert werden.

## Patentansprüche

1. Probenträger zur Untersuchung von Zellwachstum, umfassend
ein Substrat (1) mit einem Reservoir (2) mit einem Boden (3) und einer Seitenwand, wobei
das Reservoir bis zu einer Höhe der Seitenwand (4), die kleiner als die Gesamthöhe des Reservoirs ist, mit einem Trägermaterial (6) für Zellwachstum befüllt ist, und
die Seitenwand des Reservoirs auf der Höhe eine Kante (5) derart aufweist, dass ein Kontaktwinkel des Trägermaterials bezüglich der Seitenwand derart einstellbar ist, dass das Trägermaterial eine plane Oberfläche aufweist,
wobei das Trägermaterial ein Gel, insbesondere ein vernetztes oder polymerisiertes Gel, Silikon oder ein Polymer, insbesondere ein vernetztes Polymer, umfasst.

2. Probenträger nach Anspruch 1, wobei die Kante (5) eine umlaufende Kante ist.

3. Probenträger nach Anspruch 1 oder 2, wobei das Reservoir parallel zum Boden unmittelbar unterhalb der Höhe eine erste Querschnittsfläche und unmittelbar oberhalb der Höhe eine zweite Querschnittsfläche aufweist, wobei die zweite Querschnittsfläche größer als die erste Querschnittsfläche ist.

4. Probenträger nach einem der vorangegangenen Ansprüche, wobei das Reservoir parallel zum Boden vom Boden bis zu der vorherbestimmen Höhe eine erste Querschnittsfläche und von der Höhe bis zu einer weiteren Höhe eine zweite Querschnittsfläche aufweist, wobei die zweite Querschnittsfläche größer als die erste Querschnittsfläche ist.

5. Probenträger nach Anspruch 3 oder 4, wobei das Größenverhältnis von zweiter zu erster Querschnittsfläche wenigstens 1,2, vorzugsweise wenigstens 1,8, weiter bevorzugt 2 - 4, beträgt.

6. Probenträger nach einem der Ansprüche 3 - 5, wobei die erste Querschnittsfläche eine Fläche von 0,8 -175 mm², vorzugsweise von 0,8 - 65 mm², aufweist.

7. Probenträger nach einem der vorangegangenen Ansprüche, wobei die Höhe 50 µm - 1 mm, vorzugsweise 50 - 250 µm, beträgt.

8. Probenträger nach einem der vorangegangenen Ansprüche, wobei der Boden eine Dicke von 1 µm bis 2 mm, vorzugsweise 10 µm bis 250 µm, aufweist.

9. Probenträger nach einem der vorangegangenen Ansprüche, wobei der Boden ein optisch transparentes Material, insbesondere Glas oder einen Kunststoff, wie COC, COP, PMMA, PC, PE oder PS, umfasst.

10. Probenträger nach einem der vorangegangenen Ansprüche, wobei der Boden für sichtbares Licht im wesentlichen keine Eigenfluoreszenz und/oder einen Brechungsindex größer als 1,2 und/oder kleiner als 1,7 aufweist.

11. Probenträger nach einem der vorangegangenen Ansprüche, wobei der Kontaktwinkel derart eingestellt ist, dass die Differenz zwischen dem Kontaktwinkel und dem Neigungswinkel der Seitenwand bezüglich des Bodens 0° beträgt.

12. Probenträger nach einem der vorangegangenen Ansprüche, wobei die Seitenwand an Bereichen hydrophil und/oder hydrophob ausgebildet ist.

13. Probenträger nach einem der vorangegangenen Ansprüche, wobei das Substrat einen Kunststoff umfasst.

14. Probenträger nach einem der vorangegangenen Ansprüche, wobei das Substrat eine Kunststofffolie umfasst, durch die der Boden des Reservoirs gebildet wird.

15. Probenträger nach einem der Ansprüche 1 - 14, wobei das Substrat einstückig, insbesondere als Spritzgussteil, ausgebildet ist.

## Claims

1. Specimen carrier for examining cell growth, comprising a substrate (1) with a reservoir (2) having a bottom (3) and a side wall, wherein
the reservoir is filled with a carrier material (6) for cell growth up to a height of the side wall (4) which is lower than the overall height of the reservoir, and
the side wall of the reservoir has an edge (5) at the height such that a contact angle of the carrier material with respect to the side wall can be adjusted so that the carrier material exhibits a planar surface,
wherein the carrier material comprises a gel, in particular a cross-linked or polymerised gel, silicone or a polymer, in particular a cross-linked polymer.

2. Specimen carrier according to claim 1, wherein the edge (5) is a circumferential edge.

3. Specimen carrier according to either claim 1 or claim 2, wherein the reservoir has parallel to the bottom a first cross-sectional area immediately below the height and a second cross-sectional area immediately above the height, the second cross-sectional area being larger than the first cross-sectional area.

4. Specimen carrier according to any one of the preceding claims, wherein the reservoir has a first cross-sectional area parallel to the bottom from the bottom up to the predetermined height and has a second cross-sectional area from the height up to a further height, the second cross-sectional area being larger than the first cross-sectional area.

5. Specimen carrier according to either claim 3 or claim 4, wherein the size ratio of the second cross-sectional area to the first cross-sectional area is at least 1.2, preferably at least 1.8, more preferably 2 - 4.

6. Specimen carrier according to any one of claims 3 to 5, wherein the first cross-sectional area has an area of 0.8 to 175 mm², preferably 0.8 to 65 mm².

7. Specimen carrier according to any one of the preceding claims, wherein the height is 50 µm -1 mm, preferably 50 - 250 µm.

8. Specimen carrier according to any one of the preceding claims, wherein the bottom has a thickness of 1 µm to 2 mm, preferably 10 µm to 250 µm.

9. Specimen carrier according to any one of the preceding claims, wherein the bottom comprises an optically transparent material, in particular glass or a plastic such as COC, COP, PMMA, PC, PE or PS.

10. Specimen carrier according to any one of the preceding claims, wherein the bottom exhibits essentially no autofluorescence for visible light and/or a refractive index greater than 1.2 and/or less than 1.7.

11. Specimen carrier according to any one of the preceding claims, wherein the contact angle is adjusted such that the difference between the contact angle and the slope angle of the side wall with respect to the bottom is 0°.

12. Specimen carrier according to any one of the preceding claims, wherein the side wall is configured to be hydrophilic and/or hydrophobic in regions.

13. Specimen carrier according to any one of the preceding claims, wherein the substrate comprises a plastic.

14. Specimen carrier according to any one of the preceding claims, wherein the substrate comprises a plastic film which forms the bottom of the reservoir.

15. Specimen carrier according to any one of claims 1 to 14, wherein the substrate is formed from one piece, in particular as an injection moulded part.

## Revendications

1. Porte-échantillons pour l'analyse de la croissance cellulaire, comprenant
un substrat (1) avec un réservoir (2) présentant un fond (3) et une paroi latérale, étant précisé
que le réservoir est rempli d'un matériau de support (6) pour la croissance cellulaire jusqu'à une hauteur de la paroi latérale (4) qui est inférieure à la hauteur totale du réservoir, et
que la paroi latérale du réservoir présente sur ladite hauteur un bord (5) de telle sorte qu'un angle de contact du matériau de support soit réglable par rapport à la paroi latérale pour que le matériau de support présente une surface plane,
le matériau de support comprenant un gel, en particulier un gel réticulé ou polymérisé, une silicone ou un polymère, en particulier un polymère réticulé.

2. Porte-échantillon selon la revendication 1, étant précisé que le bord (5) est un bord circulaire.

3. Porte-échantillon selon la revendication 1 ou 2, étant précisé que le réservoir présente parallèlement au fond, juste au-dessous de ladite hauteur, une première surface de section transversale, et juste au-dessus de ladite hauteur, une seconde surface de section transversale, la seconde surface de section transversale étant plus grande que la première.

4. Porte-échantillon selon l'une des revendications précédentes, étant précisé que le réservoir présente parallèlement au fond, du fond jusqu'à la hauteur définie préalablement, une première surface de section transversale et, de ladite hauteur jusqu'à une autre hauteur, une seconde surface de section transversale, la seconde surface de section transversale étant plus grande que la première.

5. Porte-échantillon selon la revendication 3 ou 4, étant précisé que le rapport de taille de la seconde sur la première surface de section transversale est d'au moins 1,2, de préférence d'au moins 1,8, plus spécialement de 2-4.

6. Porte-échantillon selon l'une des revendications 3 à 5, étant précisé que la première surface de section transversale présente une surface de 0,8-175 mm², de préférence de 0,8-65 mm².

7. Porte-échantillon selon l'une des revendications précédentes, étant précisé que ladite hauteur est de 50 µm-1 mm, de préférence de 50 à 250 µm.

8. Porte-échantillon selon l'une des revendications précédentes, étant précisé que le fond présente une épaisseur de 1 µm à 2 mm, de préférence de 10 µm à 250 µm.

9. Porte-échantillon selon l'une des revendications précédentes, étant précisé que le fond comprend un matériau optiquement transparent, en particulier du verre ou une matière plastique telle que COC, COP, PMMA, PC, PE ou PS.

10. Porte-échantillon selon l'une des revendications précédentes, étant précisé que le fond ne présente pratiquement pas de fluorescence propre pour la lumière visible et/ou présente un indice de réfraction supérieur à 1,2 et/ou inférieur à 1,7.

11. Porte-échantillon selon l'une des revendications précédentes, étant précisé que l'angle de contact est réglé de telle sorte que la différence entre l'angle de contact et l'angle d'inclinaison de la paroi latérale par rapport au fond soit de 0°.

12. Porte-échantillon selon l'une des revendications précédentes, étant précisé que la paroi latérale est conçue pour être hydrophile et/ou hydrophobe au niveau de certaines zones.

13. Porte-échantillon selon l'une des revendications précédentes, étant précisé que le substrat comprend une matière plastique.

14. Porte-échantillon selon l'une des revendications précédentes, étant précisé que le substrat comprend un film de matière plastique grâce auquel est formé le fond du réservoir.

15. Porte-échantillon selon l'une des revendications 1 à 14, étant précisé que le substrat est conçu d'une seule pièce, en particulier sous la forme d'une pièce moulée par injection.
